# EUROPEAN PATENT SPECIFICATION

(11) **EP 0 529 010 B1**
(45) Date of publication and mention of the grant of the patent: **06.09.1995**
(21) Application number: 91920970.0
(22) Date of filing: 30.04.1991
(51) Int. Cl.: C12N 15/62, C12N 5/00

(54) **MAMMALIAN CELLS EXPRESSING A HYBRID RECEPTOR**
EINEN HYBRIDEN REZEPTOR EXPRIMIERENDE SÄUGERZELLEN
CELLULES DE MAMMIFERES EXPRIMANT UN RECEPTEUR HYBRIDE

(30) Priority: 30.04.1990 DK 1064/90
(43) Date of publication of application: 03.03.1993
(73) Proprietor: NOVO NORDISK A/S, 2880 Bagsvaerd (DK)
(72) Inventor: WIBERG, Finn, Christoph, DK-3520 Farum (DK); OLSEN, Frank, DK-2800 Lyngby (DK); BOEL, Esper, DK-2840 Holte (DK); KJELDSEN, Thomas, Borglum, DK-2830 Virum (DK); MOLLER, Niels, Peter, Hundahl, DK-2800 Lyngby (DK)
(86) International application number: DK9100116
(87) International publication number: WO9117253

(56) References cited:
- EP-A- 0 244 221
- EP-A- 0 325 262
- WO-A-89/05355
- Proc. Natl. Acad. Sci. USA, Vol. 83, November 1986, L. ELLIS et al.: "Linking functional domains of the human insulin receptor with the bacterial aspartate receptor", see page 8137 - page 8141.
- The Journal of Cell Biology, Vol. 109, November 1989, L. SISTONEN et al.: "Activation of the neu Tyrosine Kinase Induces the fos/jun Transcription Factor Complex, the Glucose Transporter, and Ornithine Decarboxylase", see page 1911 - page 1919.
- Science, Vol. 240, June 1988, B.K. KOBILKA et al.: "Chimeric alpha2-, beta2-Adrenergic Receptors: Delineation of Domains Involved in Effector Coupling and Ligand Binding Specificity", see page 1310 - page 1316.
- Proc. Natl. Acad. Sci. USA, Vol. 84, August 1987, L. ELLIS et al.:"Heterologous transmembrane signaling by a human insulin receptor-v-ros hybridin Chinese hamster ovary cells", see page 5101 - page 5105.
- Lax et al.(1989), EMBO J., 8(2), p.421-427.

## Description

The present invention relates to a cell expressing a hybrid between different cellular receptors and a process of producing a desired polypeptide from the cell.

Cell culture has been carried out for many years in order to invenstigate for instance the physiology of mammalian cells and the relationship between mammalian cells and other organisms such as viruses as well as the impact of a large number of different compounds on the cells. The equipment tranditionally used for cell culture includes glass vessels, petri dishes, T-flasks and the like. The media formulations used for cell culture experiments have been named for the persons who developed them (often on a trial-and-error basis) or modified them in various ways, i.a. Ham, Dulbecco, Leibovits, McCoy, Waymouth, Eagle, Iscove, etc.

These conventional media all contain serum which was found to make the cells grow at a reasonable rate for experimentation purposes, and the serum which has generally been found to provide the best growth rate is foetal calf serum. The scarcity and variable quality of this serum has created serious problems for cell culture in that the experiments have often been difficult to reproduce.

Although other aspects of cell culture, in particular the equipment used to culture the cells on a larger scale, have undergone a rapid development in recent years, the composition of the standard media used for cultivation is substantially the same as in the early days of cell culture. Thus, the media still have a high serum content.

Modern concepts of producing pharmaceuticals on the basis of cell culture require a stringent process control, not only with respect to purification, but also with respect to culture conditions. The use of serum in media is inconsistent with this requirement: due to its biological nature, the composition of serum varies from batch to batch resulting in variability of the cultivation process. Serum also represents the single most important source of unrelated protein present in the culture liquid, which can only be removed from the product of interest by laborious methods resulting in a lower yield of the product of interest. Moreover, serum is the single most important source of contamination of the cell culture with mycoplasma, bacteria or viruses, and costly as well as time-consuming measures have to be taken to remove these contaminants from the serum and to test for their absence.

It would therefore be highly desirable to omit serum from media used for mammalian cell culture. Attempts have been made to adapt cell lines already used in the production of pharmaceuticals or potentially useful cell lines to growth in serum-free media or media with a reduced serum content. It has, however, been observed that when adaptations to serum-free or low serum media have been made, this has been at the expense of growth performance, i.e. the cells grow at a slower rate than in serum-containing media, resulting in lower productivity and higher susceptibility to invading contaminants.

A cell line which is able to grow at an increased growth rate than normally observed in serum-containing media, not only in serum-containing media, but also in serum-free media would therefore be of great potential interest for cell culture in the pharmaceutical industry. By omitting serum, it would be simpler to purify the product of interest resulting in improved yields and purity, and a stable faster cell growth rate would lead to a high process productivity, i.a. because rapid-growing cells would be less susceptible to infectious organisms.

It has surprisingly been found that cells with favourable growth properties in serum-free media may be obtained by introducing into the cells a DNA construct which comprises a hybrid DNA sequence coding for parts of two different cell surface receptors.

Accordingly, the present invention relates to a mammalian cell containing a hybrid DNA insert which comprises a first DNA sequence encoding part of the extracellular domain of the insulin receptor and a second DNA sequence encoding part of the extracellular domain of insulin-like growth factor (IGF) receptor, said cell having improved growth properties in serum-free culture media compared to cells which do not contain said hybrid DNA insert.

Cellular receptors which are typically located on cell surfaces mediate certain important cell to cell interactions involving the transmission of extracellular signals by the interaction with ligands. Such receptors are composed of an extracellular domain (in monomeric or dimeric form) which is capable of specifically recognizing and binding a particular ligand and which may have highly glycosylated and protease-resistant structure, a transmembrane domain which is responsible for anchoring the receptor in the cell membrane and which consists of a hydrophobic sequence of some 25 amino acids, and a cytoplasmic domain which is responsible for generating a cellular signal as a response to the binding of the ligand to the extracellular domain; the cytoplasmic domain(s) may define an enzymatic activity which is triggered by ligand binding.

The reason why hybrid insulin/IGF-1 receptors of the invention may promote the growth of cells in serum-free media has not yet been determined. It is, however, currently assumed that such hybrid receptor constructions provide a constant stimulation of the growth regulatory system of the cells so that their dependence on growth factors (e.g. those present in serum) is reduced. Furthermore, it is possible that hybrid receptors are less sensitive to non-activating ligands (e.g. degradation products or metabolites from the cells) and therefore less likely to be inhibited for binding than the natural receptor.

A number of naturally occurring receptors have previously been identified. Thus, Rubin et al., J. Immun. 135, 1985, pp. 3172-3177, describe the release of large quantities of the inter-leukin-2 receptor (IL-2-R) into the culture medium of activated T-cells. The DNA sequence coding for the insulin receptor has been published by A. Ullrich et al., Nature 313, 28 Feb. 1985, pp. 756-761, and S. Seino, Proc. Natl. Acad. Sci. USA 86, 1989, pp. 114-118. Similarly, the DNA sequence coding for the IGF-I receptor has been published by A. Ullrich et al., The EMBO J. 5(10), 1986, pp. 2503-2512.

European Patent Application, Publication No. 244 221 discloses receptors which are hybrids between the extracellular ligand-binding domain of one receptor fused to a heterologous reporter polypeptide which may be the cytoplasmic domain of another receptor.

B. Kobilka et al, Science 240, 3 June 1988, pp. 1310-1316, disclose chimeras between α2 and β2 adrenergic receptors constructed by replacing DNA sequences encoding various parts of the membrane-spanning domain of one of the receptors by the DNA sequences encoding corresponding parts of the other receptor and expressing the sequences in Xenopus laevis oocytes.

I. Lax et al., The EMBO J. 8(2), 1989, pp. 421-427, disclose chimeras between chicken and human EGF receptors constructed by replacing DNA sequences encoding various parts of one of the receptors by DNA sequences encoding the corresponding parts of the other receptor, and expressed on the surface of mammalian cells.

However, none of these prior publications discloses or suggest any use of hybrid insulin/IGF-1 receptors for promoting the growth of mammalian cells in serum-free media.

### DETAILED DISCLOSURE OF THE INVENTION

In particular, the DNA insert introduced into the cell of the present invention is one in which either the first or the second DNA sequence may encode a ligand-binding site of the insulin or IGF-1 receptor.

When producing a cell of the invention, it may therefore be an advantage to initially replace one or more exons from one of the receptors by the corresponding exon or exons from the other receptor, and to test the growth properties of cells into which the DNA construct coding for each hybrid has been introduced.

In this embodiment of the DNA insert introduced in the cell of the invention, a DNA sequence encoding one or more exons, or a fragment thereof, of the extracellular domain of the insulin receptor may be replaced by a DNA sequence encoding the corresponding exon or exons, or fragment thereof, of the extracellular domain of the IGF-1 receptor. Alternatively, a DNA sequence encoding one or more exons, or a fragment thereof, of the extracellular domain of the IGF-1 receptor may be replaced by a DNA sequence encoding the corresponding exon or exons, or fragment thereof, of the extracellular domain of the insulin receptor.

More specifically, in the DNA insert introduced in the cell of the invention, the DNA sequence coding for exon 2, or a fragment thereof, of the insulin receptor may be replaced by the DNA sequence coding for exon 2, or a fragment thereof, of the IGF receptor. Alternatively, the DNA sequence coding for exon 3, or a fragment thereof, of the insulin receptor may be replaced by the DNA sequence coding for exon 3, or a fragment thereof, of the IGF receptor. In a further alternative embodiment, the DNA sequence coding for exons 2 and 3, or a fragment thereof, of the insulin receptor may be replaced by the DNA sequence coding for exons 2 and 3, or a fragment thereof, of the IGF receptor.

In another embodiment of the DNA insert, the DNA sequence coding for exon 2, or a fragment thereof, of the IGF receptor may be replaced by the DNA sequence coding for exon 2, or a fragment thereof, of the insulin receptor. Alternatively, the DNA sequence coding for exon 3, or a fragment thereof, of the IGF receptor may be replaced by the DNA sequence coding for exon 3, or a fragment thereof, of the insulin receptor. As a further alternative, the DNA sequence coding for exons 2 and 3, or a fragment thereof, of the IGF receptor may be replaced by the DNA sequence coding for exons 2 and 3, or a fragment thereof, of the insulin receptor.

Similarly, a DNA sequence coding for exon 1 (preferably combined with exon 2 or a fragment thereof) or one or more of exons 4-11 or fragments thereof of the insulin receptor may be replaced by the corresponding DNA sequence from the IGF receptor, or vice versa.

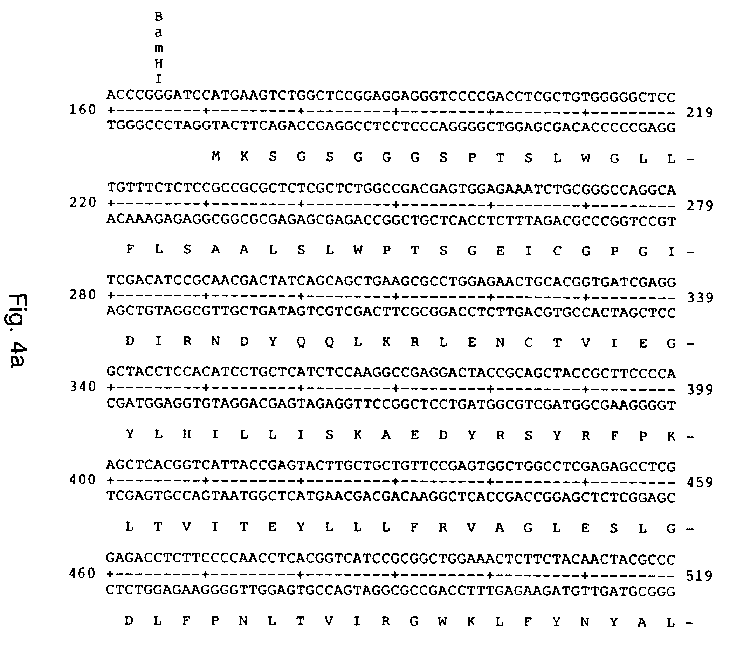

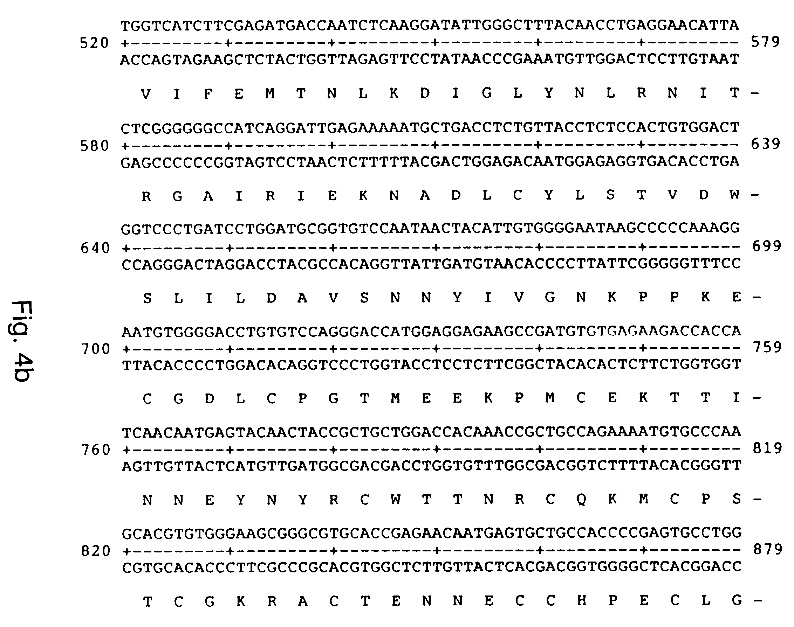

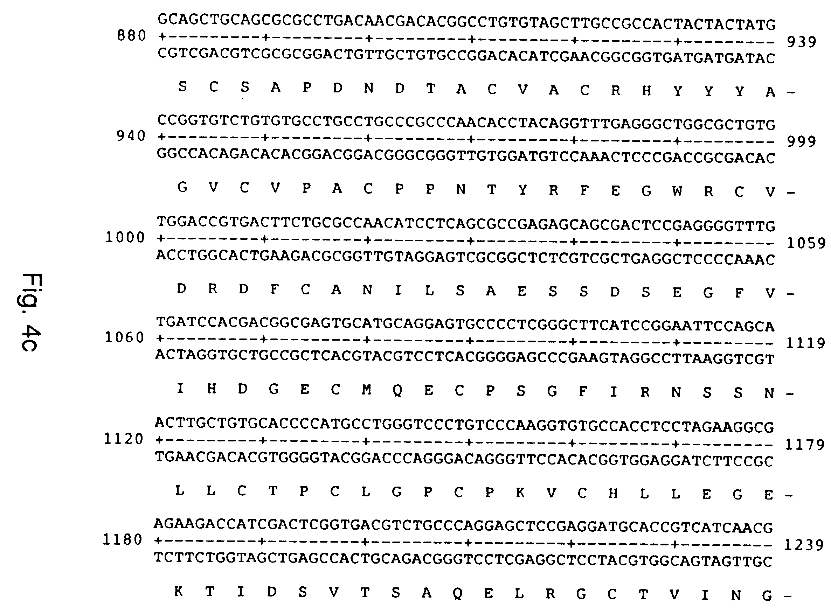

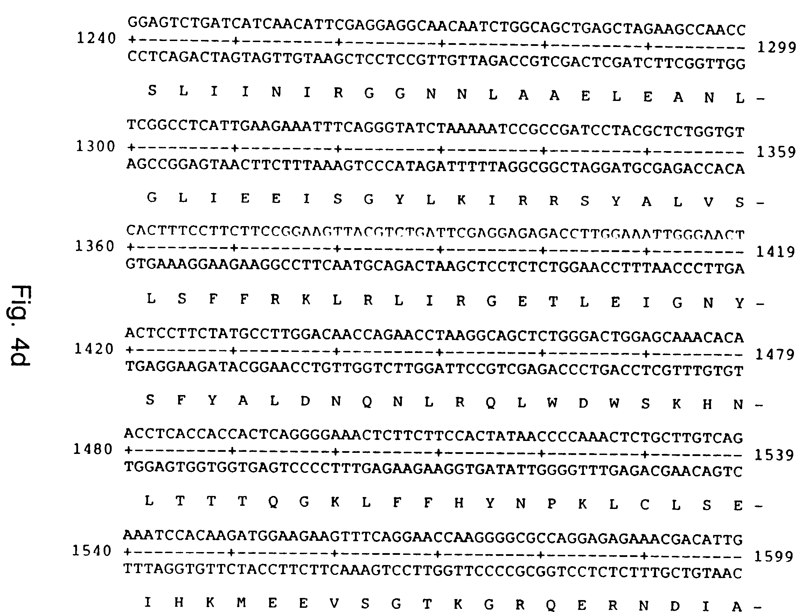

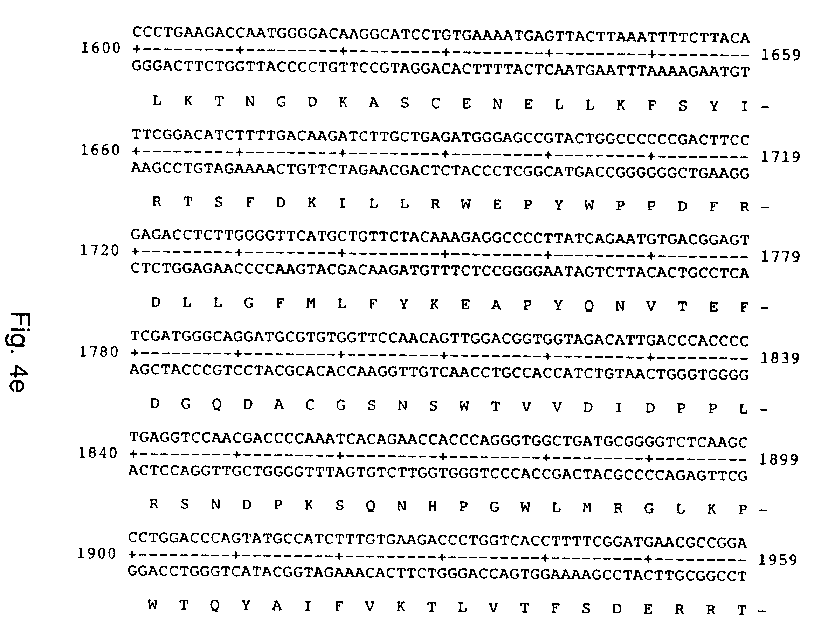

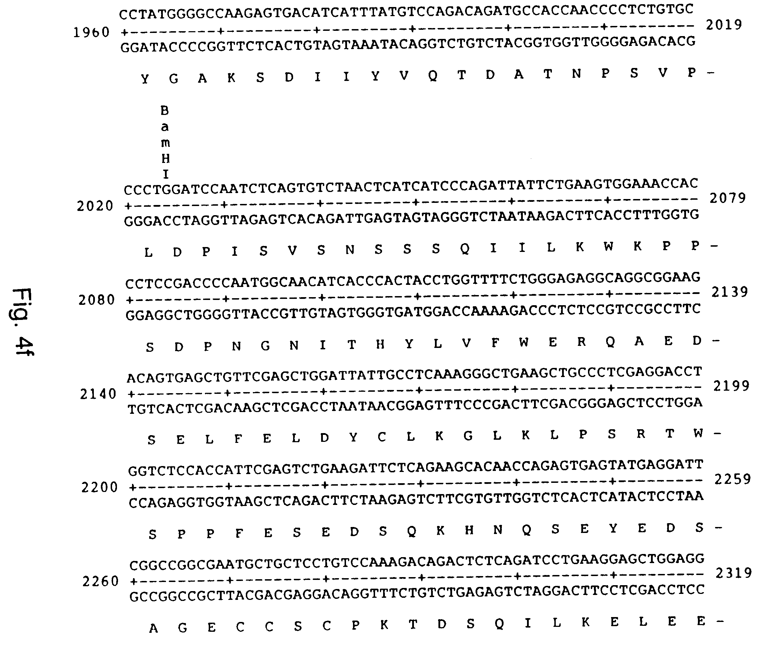

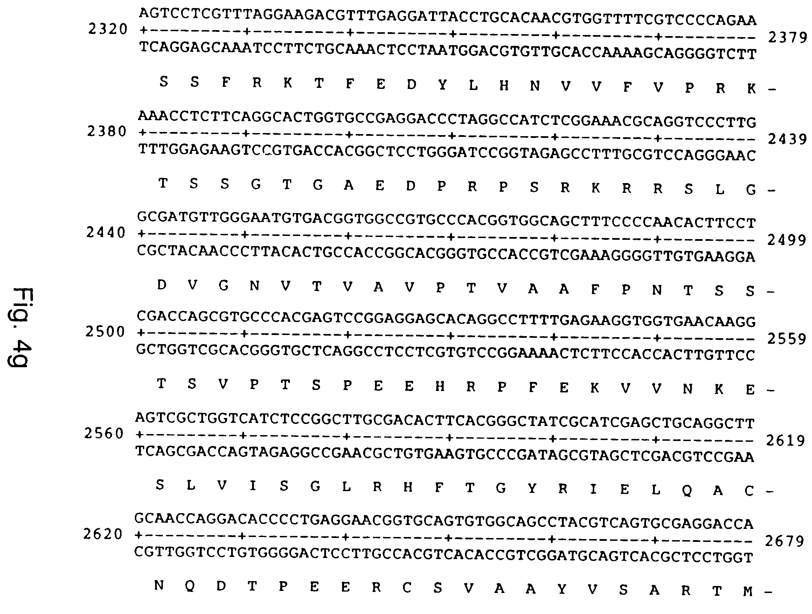

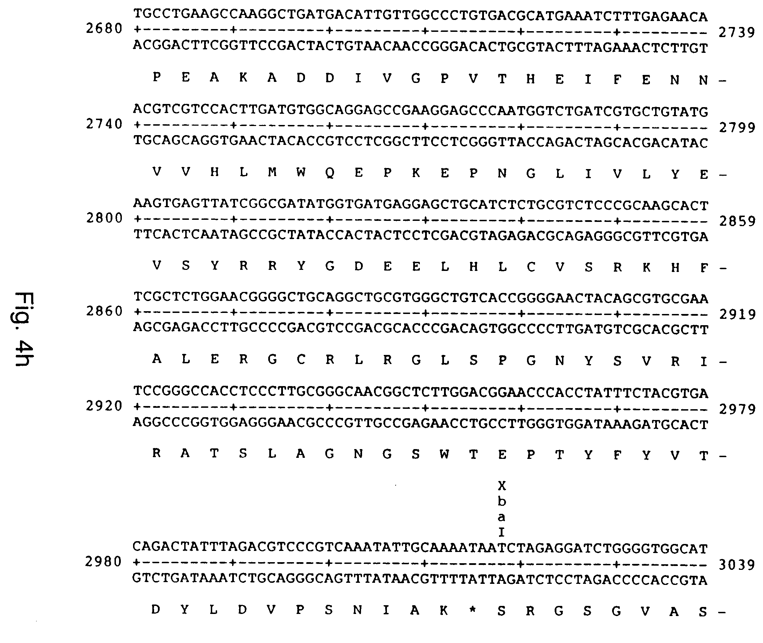

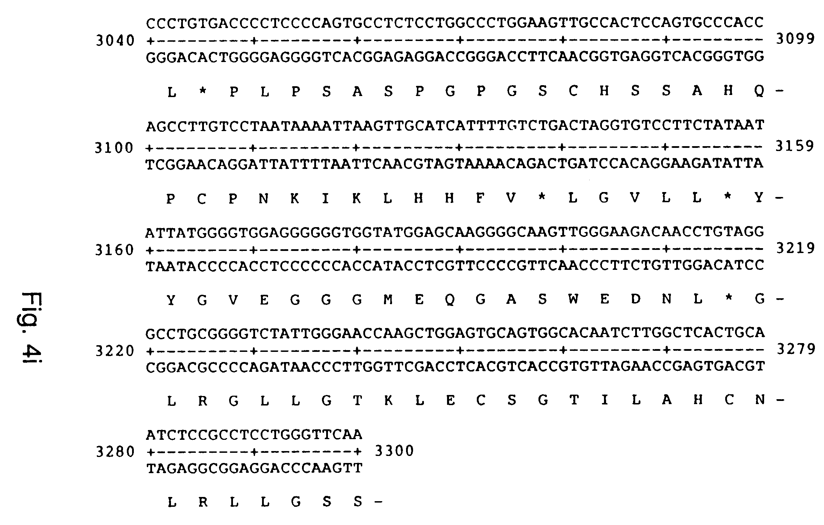

One example of a cell of the invention exhibiting favourable growth properties is one in which a DNA insert has been introduced, which has the partial DNA sequence shown in Fig. 4A-4F (encoding the extracellular domain of the hybrid receptor), or a suitable modification thereof. Suitable modifications of the DNA sequence may comprise nucleotide substitutions which do not give rise to another amino acid sequence of the hybrid polypeptide, but which facilitate the production of the polypeptide, or nucleotide substitutions which do give rise to a different amino acid sequence of the hybrid polypeptide. Other possible modifications may be insertion of one or more nucleotides into the sequence, addition of one or more nucleotides at either end of the sequence and deletion of one or more nucleotides at either end of or within the sequence.

The DNA insert encoding the hybrid receptor may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

The DNA insert may also be of genomic or cDNA origin, for instance obtained by preparing a genomic or cDNA library and screening for DNA sequences coding for all or part of the hybrid receptor polypeptide by hybridization using synthetic oligonucleotide probes in accordance with standard techniques (cf. Sambrook et al., Molecular Cloning: A Laboratory Manual, 2nd Ed., Cold Spring Harbor, 1989). In this case, a genomic or cDNA sequence encoding a part of either receptor may be modified at a site corresponding to the site(s) at which it is desired to introduce amino acid substitutions, e.g. by site-directed mutagenesis using synthetic oligonucleotides encoding the desired amino acid sequence for homologous recombination in accordance with well-known procedures.

Finally, the DNA insert may be of mixed synthetic and genomic, mixed synthetic and cDNA or mixed genomic and cDNA origin prepared by annealing fragments of synthetic, genomic or cDNA origin (as appropriate), the fragments corresponding to various parts of the entire DNA insert, in accordance with standard techniques. The DNA insert may also be prepared by polymerase chain reaction using specific primers, for instance as described in US 4,683,202.

The hybrid DNA insert may be introduced into the cell by transfection of the cell with a recombinant expression vector comprising the insert. The expression vector may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the hybrid DNA insert should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA insert in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter.

The DNA insert should also be operably connected to a suitable terminator, such as the human growth hormone terminator (Palmiter et al., op. cit.). The vector may further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector may also comprise a DNA sequence enabling the vector to replicate in the host cell in question. An examples of such a sequence is the SV 40 origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hygromycin or methotrexate.

The procedures used to ligate the hybrid DNA insert with the DNA sequences coding for the promoter and the terminator, respectively, and to insert them into suitable vectors containing the information necessary for replication, are well known to persons skilled in the art (cf., for instance, Sambrook et al., op.cit.).

The cell of the present invention may suitably be used for the production of proteins of polypeptides of interest. Thus, the cell may further contain an inserted DNA sequence encoding a desired polypeptide. Examples of such polypeptides are the human blood clotting factors IX, VIII or VII, human tissue-type plasminogen activator, human protein C, human plasminogen, etc.

Examples of suitable mammalian cells for transfection with expression vector containing the DNA insert described above are the COS (ATCC CRL 1650), BHK (ATCC CRL 1632, ATCC CCL 10) NIH/3T3 (ATCC CRL 1658) or CHO (ATCC CCL 61) cell lines. Methods of transfecting mammalian cells and expressing DNA sequences introduced in the cells are described in e.g. Kaufman and Sharp, J. Mol. Biol. 159, 1982, pp. 601-621; Southern and Berg, J. Mol. Appl. Genet. 1, 1982, pp. 327-341; Loyter et al., Proc. Natl. Acad. Sci. USA 79, 1982, pp. 422-426; Wigler et al., Cell 14, 1978, p. 725; Corsaro and Pearson, Somatic Cell Genetics 7, 1981, p. 603, Graham and van der Eb, Virology 52, 1973, p. 456; and Neumann et al., EMBO J. 1, 1982, pp. 841-845.

In another aspect, the present invention relates to a process for producing a desired polypeptide, which comprises culturing a cell as described above in a suitable nutrient medium under conditions which are conducive to the expression of the polypeptide, and recovering the polypeptide from the culture. Although the medium used to culture the cells may be any conventional medium suitable for growing mammalian cells, the cell of the invention has surprisingly been found to exhibit exceptionally favourable growth properties in serum-free medium which, as indicated above, is an advantage, i.a. because the purification of the desired polypeptide will be simplified, and the yield of the polypeptide will be improved.

The polypeptide produced by the cells in this manner will often be secreted to the growth medium and may be recovered from the medium by conventional procedures including separating the host cells from the medium by centrifugation or filtration, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

### BRIEF DESCRIPTION OF THE DRAWINGS

The invention is further described with reference to the drawings, wherein
Fig. 1A-1I shows the full-length cDNA sequence of the human insulin receptor;
Fig. 2 shows the assembly into the α-subunit and part of the β-subunit of the IGF-I receptor (sIGF-I-R) of cDNA fragments generated by reverse transcription and PCR using specific oligonucleotide primers; the circled numerals refer to the primers listed in Example 1;
Fig. 3A-3H shows the cDNA sequence of the soluble IGF-I receptor and the deduced amino acid sequence thereof, indicated in the conventional one-letter code;
Fig. 4A-4I shows the cDNA sequence of the extracellular domain of a hybrid insulin/IGF-I receptor, wherein exons 2 and 3 of the insulin receptor have been replaced by exons 2 and 3 of the IGF-I receptor, and the deduced amino acid sequence thereof, indicated in the conventional one-letter code;
Fig. 5 is a graph showing the growth curves (day 3-7) of three different cell lines, BHK, FCW 38-16 (BHK transfected with DNA encoding the hybrid insulin/IGF-I receptor according to Example 1) and hIR 12-14 (BHK transfected with DNA coding for the wild-type human insulin receptor), in medium containing 5% foetal calf serum;
Fig. 6 is a graph showing the growth curves (day 1-3) of three different cell lines, BHK, FCW 38-16 (BHK transfected with DNA encoding the hybrid insulin/IGF-I receptor according to Example 1) and hIR 12-14 (BHK transfected with DNA coding for the wild-type human insulin receptor), in medium containing 5% foetal calf serum; and

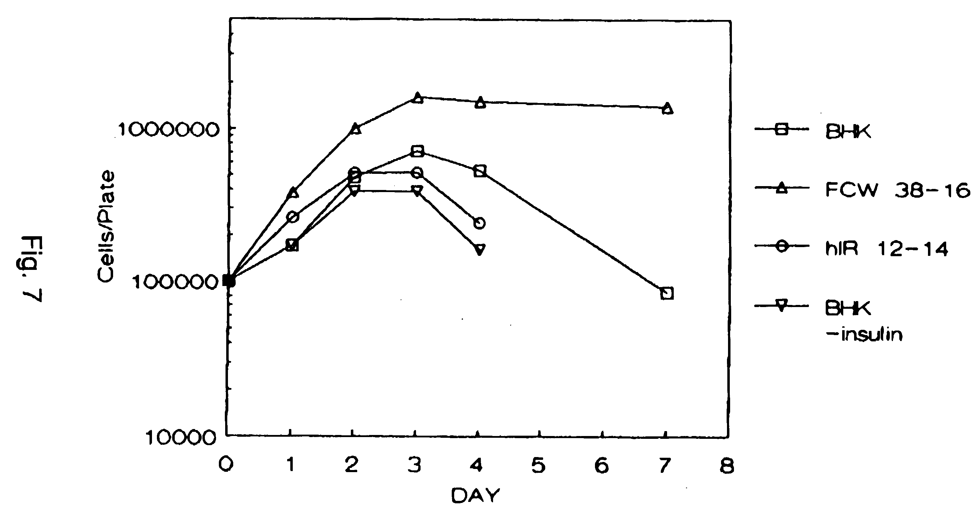

Fig. 7 is a graph showing the growth curves (day 1-8) of three different cell lines, BHK, FCW 38-16 (BHK transfected with DNA encoding the hybrid insulin/IGF-I receptor according to Example 1) and hIR 12-14 (BHK transfected with DNA coding for the wild-type human insulin receptor), in serum-free medium supplemented with human insulin (BHK cells were also grown in serum-free medium without any added human insulin).

The invention is further illustrated in the following examples which are not in any way intended to limit the scope of the present invention.

### EXAMPLE 1

### Construction of cDNA encoding the human insulin receptor

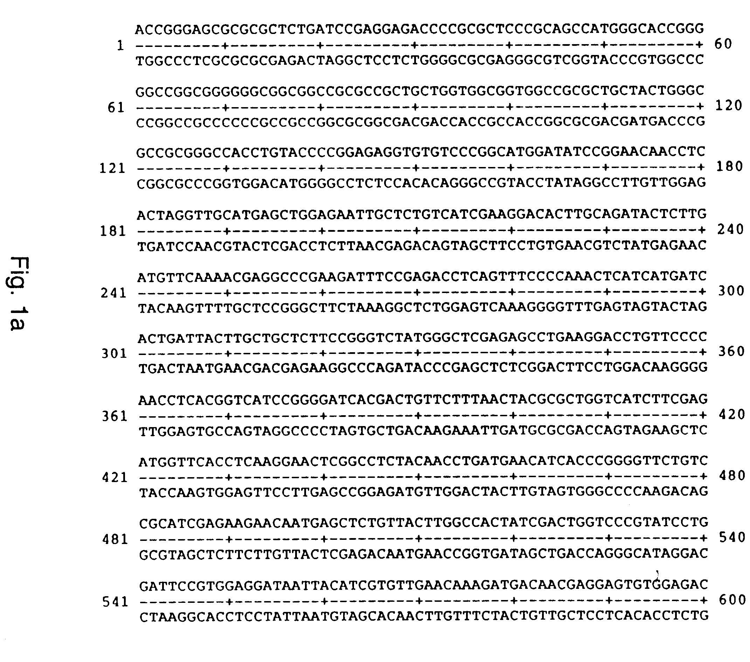

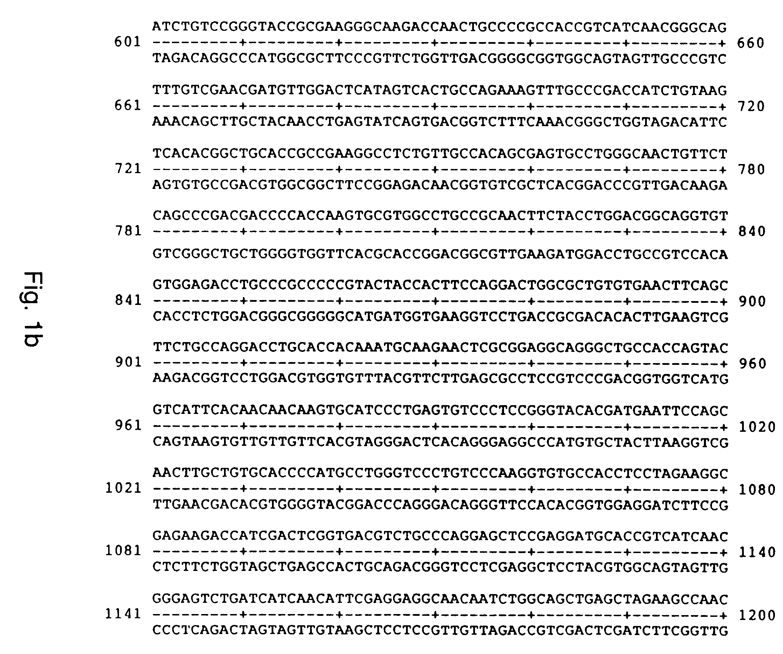

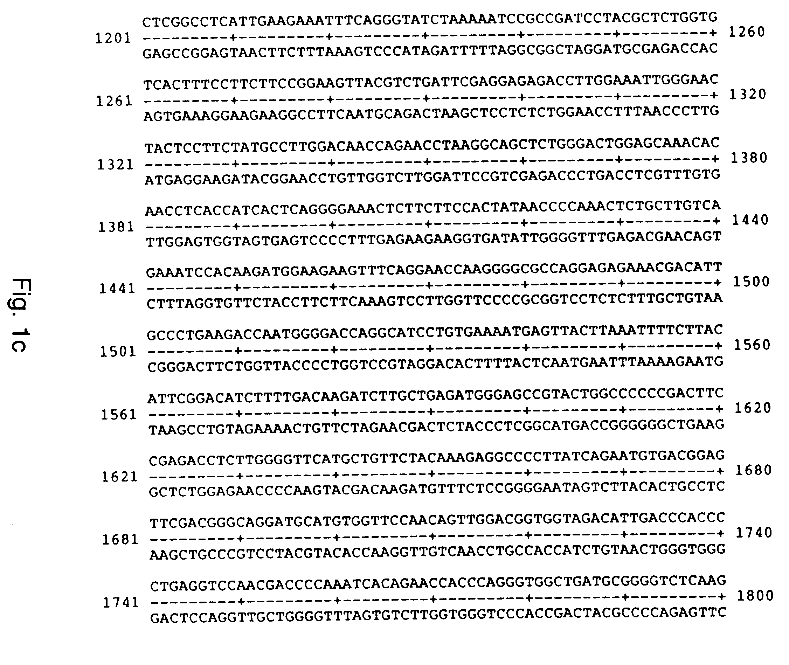

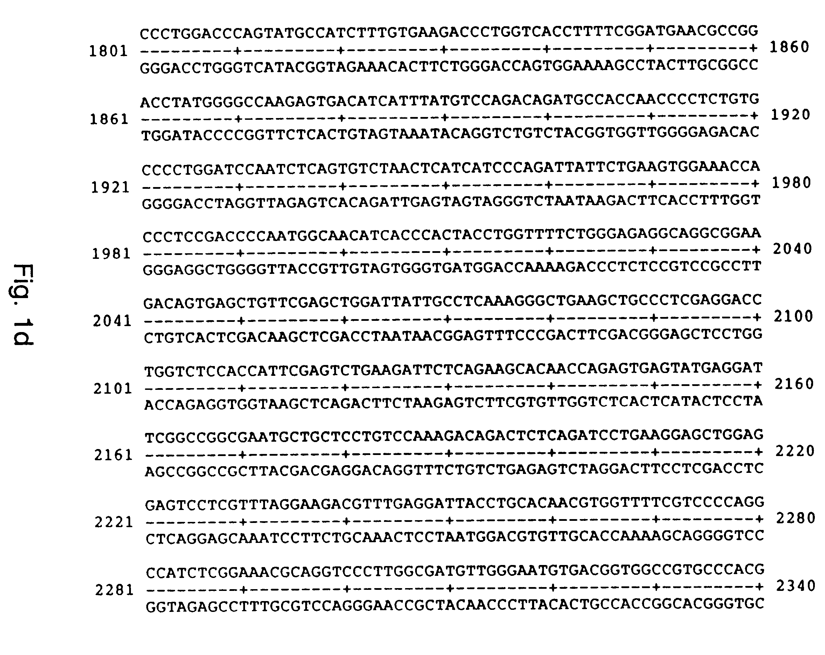

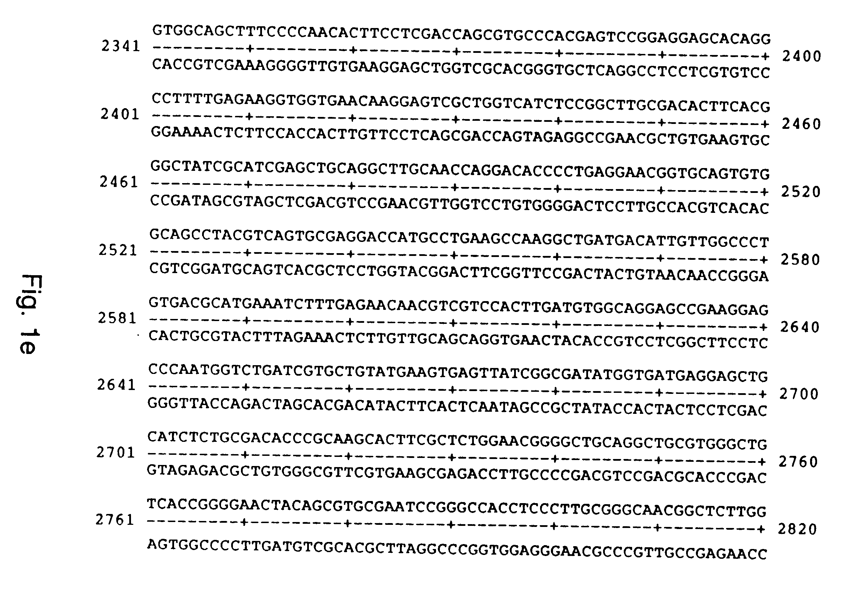

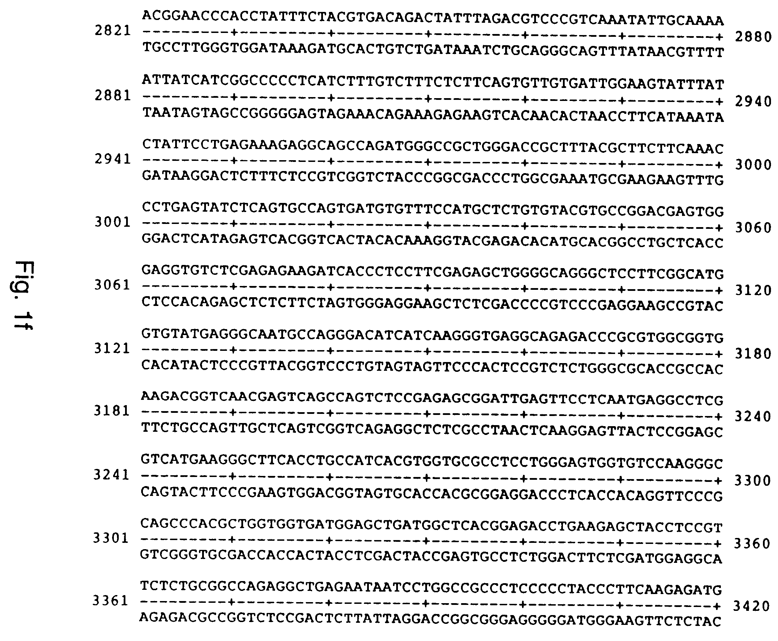

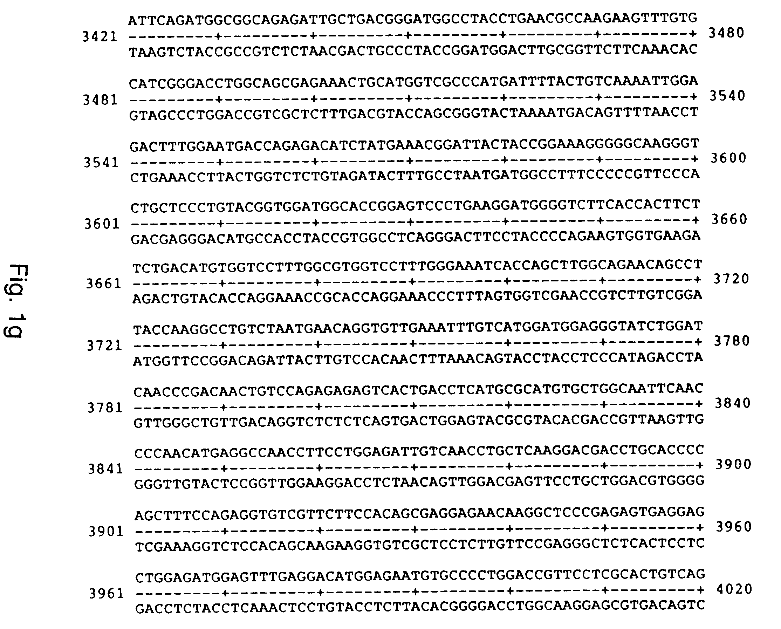

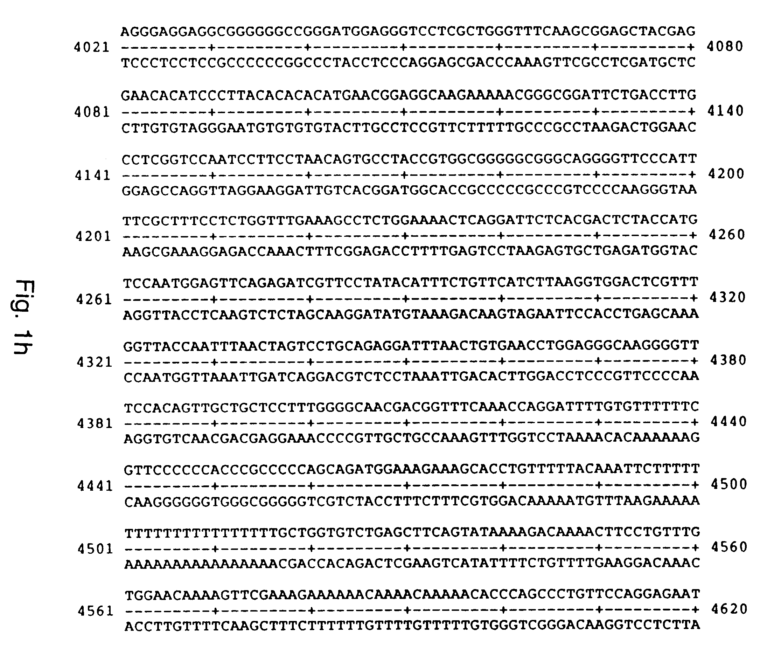

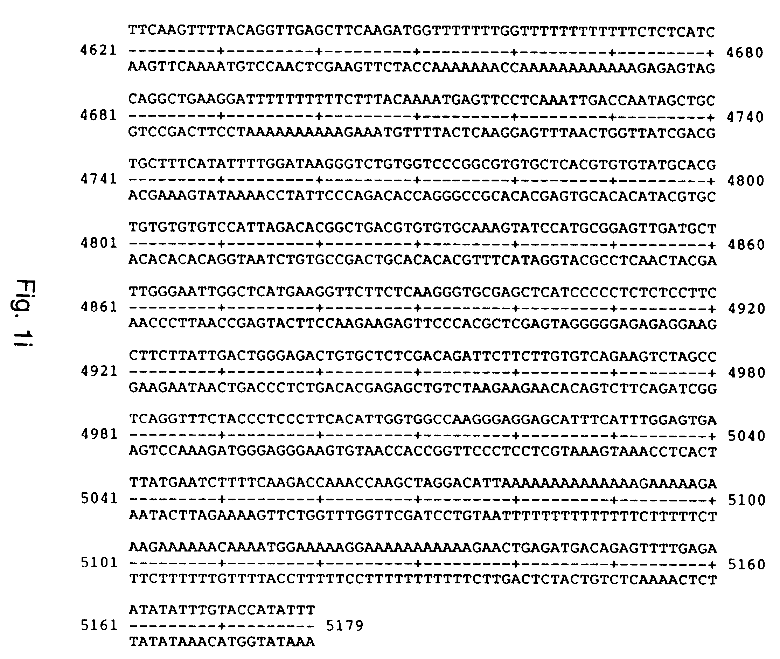

Insulin receptor cDNA was isolated from a cDNA library generated from poly(A)⁺ mRNA isolated from the human lymphoblastoid cell line IM9 (available from the American Type Culture Collection, Rockville, Maryland, under the catalogue number ATCC CCL 159) stimulated with 1.4 µg/ml cortisol for 20 hours, substantially according to the method described by Okayama and Berg, Mol. Cell Biol. 2, 1982, p. 161 ff.; Okayama and Berg, Mol. Cell. Biol. 3(2), Feb. 1983, pp. 280-289; and Noma et al., Nature 319, 1986, p. 640 ff. An approximately 4000 bp clone containing the 3'end of the insulin receptor was isolated. To obtain a full-length clone, primer extension was applied on mRNA from the IM9 cells using (10 µg total mRNA) AMV reverse transcriptase (60 U, available from Pharmacia LKB Biotechnology, Sweden) with 800 ng of the 3' oligonucleotide primer 5'-CATCTCAGCAAGATCTTGTCA-3'. The second strand was synthesized as described by Okayama and Berg, op. cit., using the 5' oligonucleotide 5'-ACCGGGAGCGCGCGCTCTGATC-3' as primer. The cDNA was digested with the restriction endonucleases BssHII and BglII and ligated into an appropriate vector. A 1550 bp clone containing the 5'end of the insulin receptor was isolated. The full-length insulin receptor was assembled in the mammalian expression vector Zem219b (described in DK Patent Application No. 3023/88) digested with NcoI and XbaI by ligating the 5' cDNA clone digested with NcoI and BglII and the 3' cDNA clone digested with SpaI and BglII. The DNA sequence of the full length insulin receptor cDNA is shown in Fig. 1A-1F.

### Construction of cDNA encoding a soluble IGF-I receptor

A soluble IGF-I receptor cDNA was prepared from mRNA from human term placenta by a method involving polymerase chain reaction (PCR) using specific oligonucleotide primers (cf. R.K. Saiki et al., Science 239, 29 Jan., 1988, pp. 487-491, and US 4,683,202). mRNA was prepared by standard procedures as described in Sambrook et al., op. cit. The mRNA was reverse transcribed into cDNA with AMV reverse transcriptase (Pharmacia LKB Biotechnology, Sweden) using the appropriate specific primers or an oligo-dT-primer at a final concentration of 800 ng/3.5 µg mRNA.

IGF-I receptor cDNA fragments were amplified by PCR using the Gene Amp kit (Perkin Elmer Cetus, Norwalk, CT, USA) as recommended by the manufacturer.

In each PCR, approximately 1µg of the reverse transcribed mRNA was used as template. The primers used for PCR all contained an endonuclease restriction site permitting subcloning and assembly of the soluble IGF-I receptor. the following specific primers were used for PCR
1. 5'-CCA AAT AGG ATC CAT GAA CTC TGG CTC CGG AGG-3'
2. 3'-CCC GAA GTA GGC CTT AAG GTC GGT CTC GT-5'
3. 5'-TGG GCA GCT GCA GCG CGC CTG-3'
4. 3'-ATT GGA CCG TGG CCA TGG CCG-5'
5. 5'-TAA CCT GGC ACC GGT ACC GGC-3'
6. 3'-TCA AAG AGT TGC TTC GAA GAC-5'
7. 5'-AAC ACC ACG GCC GCA GAC ACC-3'
8. 3'-TGT CCT ATA CTT TTG ATT AGA TCT GAC TAG T-5'

The following restriction sites native to the IGF-I receptor were part of the PCR primers: PstI, Asp718, XmaIII and HindIII. A BamHI site was incorporated in the primer 1 used to generate the 5' end of the receptor cDNA by introducing a mismatch in the sequence. The primer 8 used to generate the 3' end of the soluble IGF-I receptor cDNA was designed by introducing a mismatch in the sequence so as to include a termination codon in nucleotide position 2842-2844 followed by an XbaI site. Each PCR reaction cycle comprised denaturation of the template at 94°C for 1 minute, and annealing of the primers to the templates for 2 minutes at 50°C, followed by extension of the primers for 3 minutes at 72°C. This cycle was repeated 25 times, resulting in specific IGF-I receptor fragments.

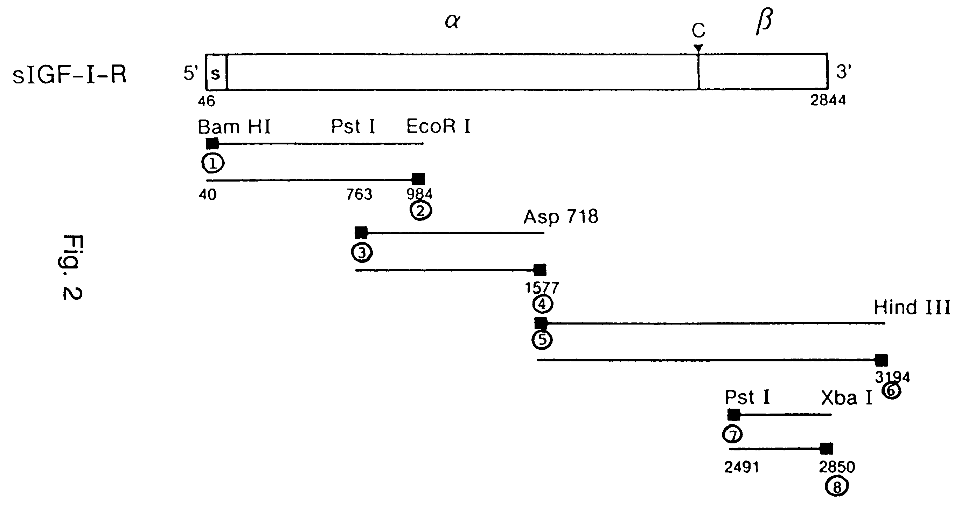

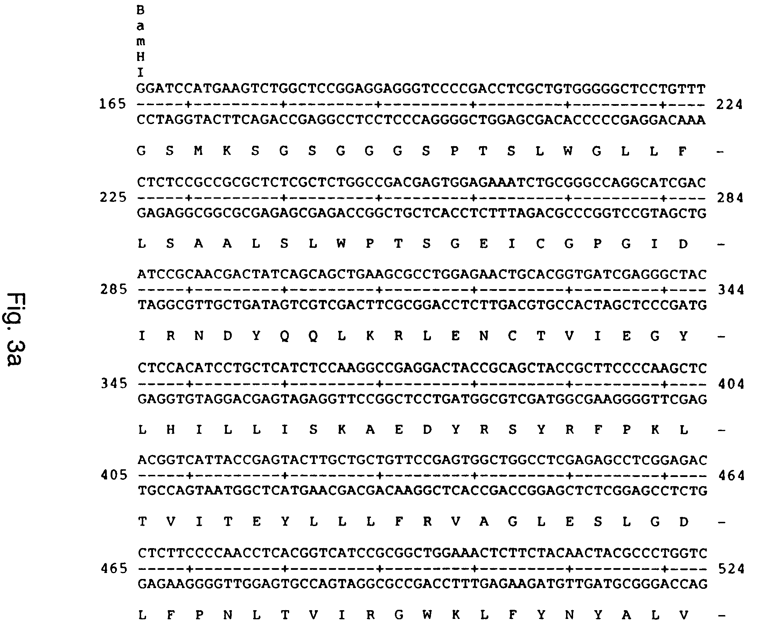

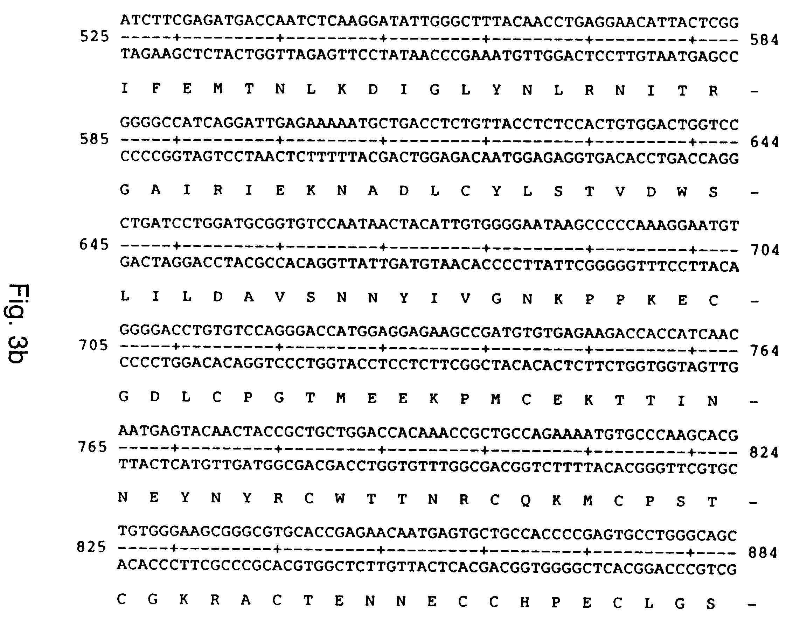

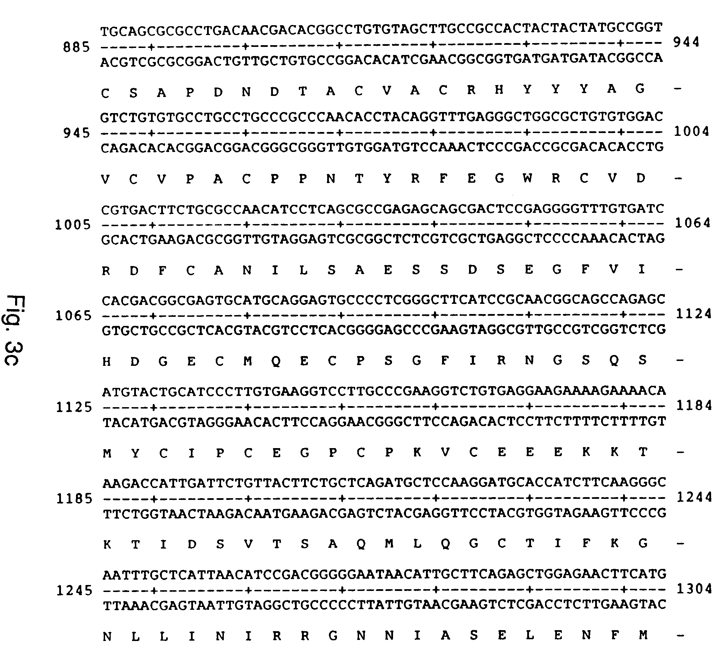

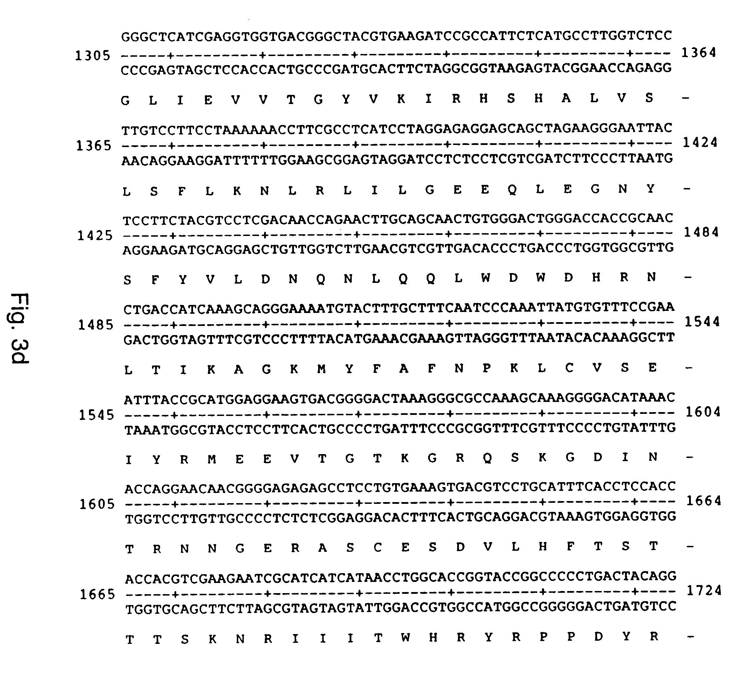

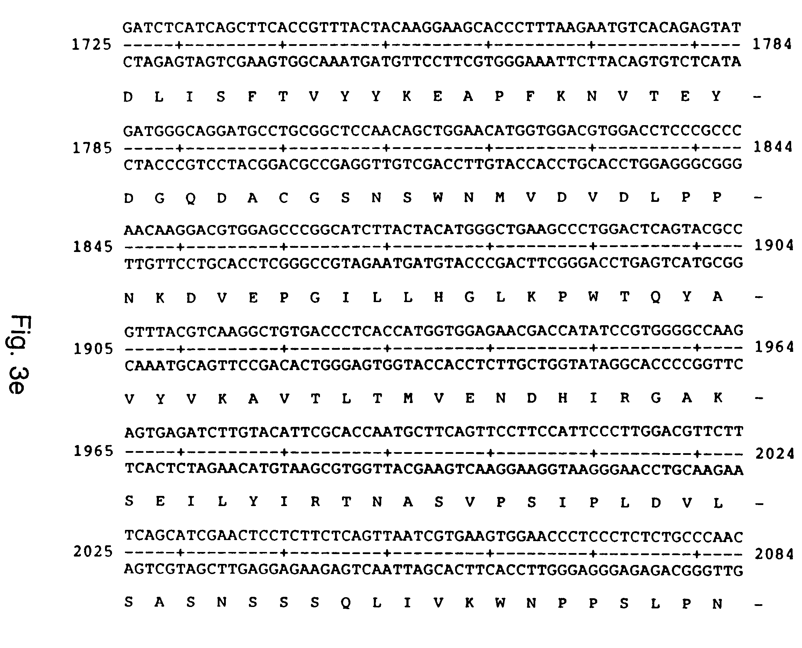

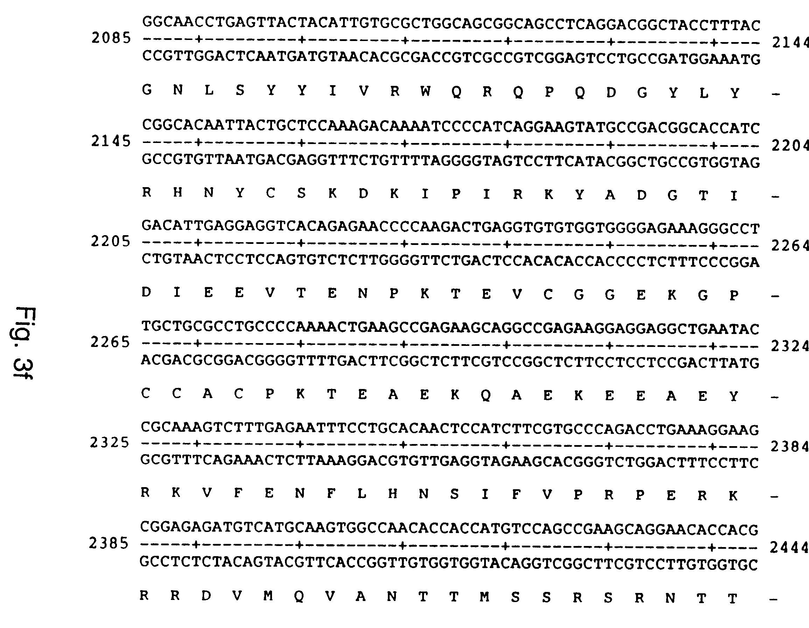

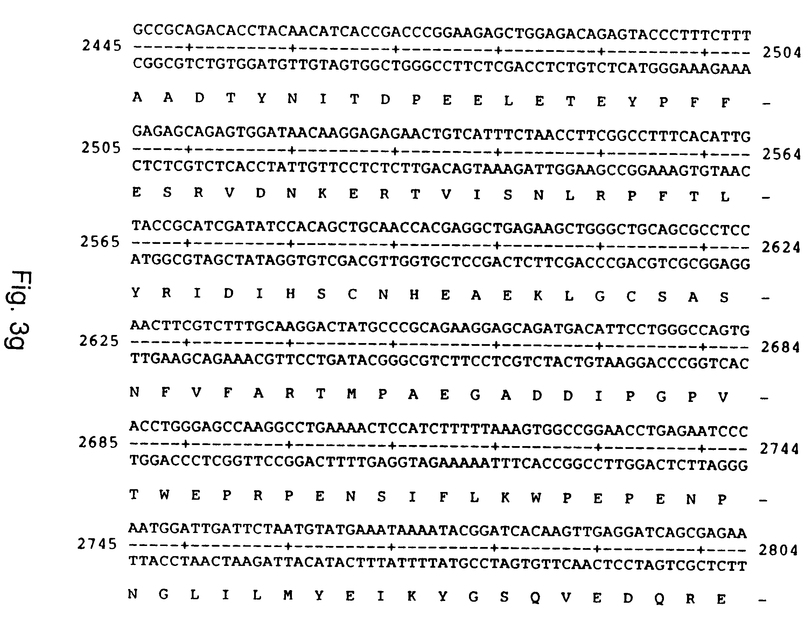

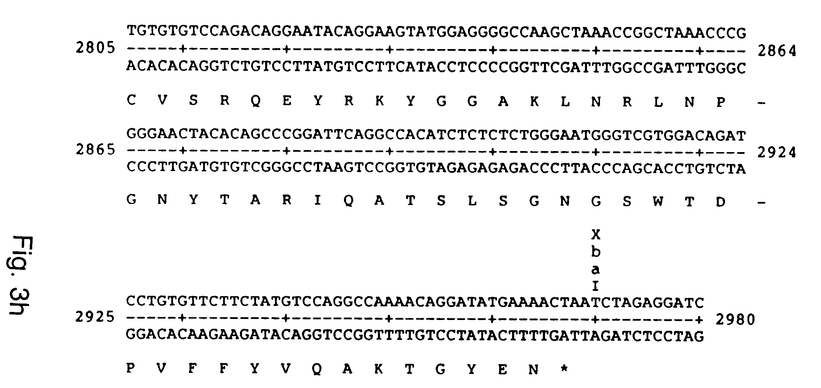

The isolated cDNA fragments were digested with the endonucleases BamHI and XbaI (New England Biolabs, MA, USA) and subcloned into the PBSII vector (Stratagene, CA, USA) by the method described by Sambrook et al., op. cit. Cells of E. coli strain MC1061 (T.V. Huynk et al., in DNA Cloning, Vol. 1 (D.M. Glover, ed.), IRL Press Ltd., Oxford, England, 1983, pp. 56-110) and SCS-1 (D. Hanahan, J. Mol. Biol. 166, 1983, pp. 557-580) were made competent according to the method described by D. Hanahan, in DNA Cloning, Vol. 1, supra, pp. 110-135, and used for transformation with the vectors indicated above. The soluble IGF-I receptor cDNA was assembled from four subcloned cDNA fragments as shown in Fig. 2 (BamHI-PstI, PstI-Asp718, Asp718-PstI, PstI-XbaI) according to the method described by Sambrook et al., op. cit. The cDNA fragment(s) were sequenced by the enzymatic chain termination method described by F. Sanger et al., op. cit., using T4 DNA polymerase (Sequenase Kit, USB, Cleveland, Ohio, USA). The cDNA sequence of the soluble IGF-I receptor is shown in Fig. 3A-3E. The sequence was identical to the published sequence (A. Ullrich et al., The EMBO Journal 5(10), 1986, pp. 2503-2512).

### Construction of cDNA coding for a hybrid insulin-IGF-I receptor

cDNA coding for a hybrid receptor composed of the human insulin receptor described above wherein the cDNA sequence coding for exons 2 and 3 was replaced by the cDNA sequence coding for exons 2 and 3 from the IGF-I receptor, was prepared by PCR as described above. Primer 2 (shown above), which includes the insulin receptor "compatible" restriction site EcoRI, was used to generate an IGF-I receptor cDNA fragment corresponding to the DNA sequence encoding exon 2 and 3 and the coding part of exon 1 of the insulin receptor which was inserted into the EcoRI site of the insulin receptor cDNA by the method described by Sambrook et al., op. cit.. The sequence of the cDNA fragment was established as described above, and the sequence of the IGF-I receptor cDNA fragment inserted into the insulin receptor was found to be identical with the published sequence (A. Ullrich et al., op. cit.). The cDNA sequence of the hybrid receptor is shown in Fig. 4A-4F.

The cDNA fragment encoding the hybrid insulin-IGF-I receptor constructed as described above was inserted in the mammalian expression vector Zem219b (described in DK Patent Application No. 3023/88) which carries a gene conferring methotrexate resistance to the host cell, and cDNA encoding the hybrid receptor was transfected into BHK cells, resulting in the cell line FCW 38-16. Resistant cells were selected with 0.4-2 µM methotrexate.

The hybrid receptor (sIGF-I-R.1-68) comprising an IGF-I receptor in which the 68 N-terminal amino acids of the insulin receptor α-subunit have replaced the equivalent IGF-I receptor segment was constructed by means of restriction enzyme sites similarly positioned in the two receptor cDNAs. The BamHI-XhoI cDNA fragments were exchanged between the insulin receptor and the IGF-I receptor resulting in sIGF-I-R.1-68. This hybrid cDNA was inserted into the mammalian expression vector pZem 219b and transfected into BHK cells, resulting in the cell line FCW 110.

During cultivation in 5% FCS, the cell line FCW 110 formed grape-like clusters of loosely attached cells, forming many floating cells that stayed alive, indicating that this cell line may be adapted to grow in suspension with or without serum.

### EXAMPLE 2

**Growth performance of cells transfected with the hybrid insulin/IGF-I receptor**

### Materials and methods

- Cell lines:: BHK (Baby Hamster Kidney cells, Syrian Hamster) FCW 38-16, according to Example 1,
hIR 12-14, BHK transfected with DNA coding for the wild-type human insulin receptor.

Cells were grown in 1 µM MTX (only the transformed cell lines), DMEM, 10% FCS, 1% PS, 1% Gln.

In a first experiment, 10⁴ cells from each cell line were divided among 25 different 5 cm culture dishes and grown in 5 % FCS on day 0. The cells were counted from 4 different plates every day. Two independent aliquots were sampled from each plate; where appropriate, floating or loosely attached cells were also counted in aspirated medium. Cells grown in this growth curve experiment were propagated in standard medium for growing mammalian cells.

In a second experiment, 10⁵ cells from each cell line were divided among 25 different 5 cm culture dishes on day 0. The medium was changed on day 1 to 0% FCS, but was supplemented with 5 mg/l of human insulin. The cells were counted over the next days as described above.

### Results

### a) Growth in medium with 5% FCS

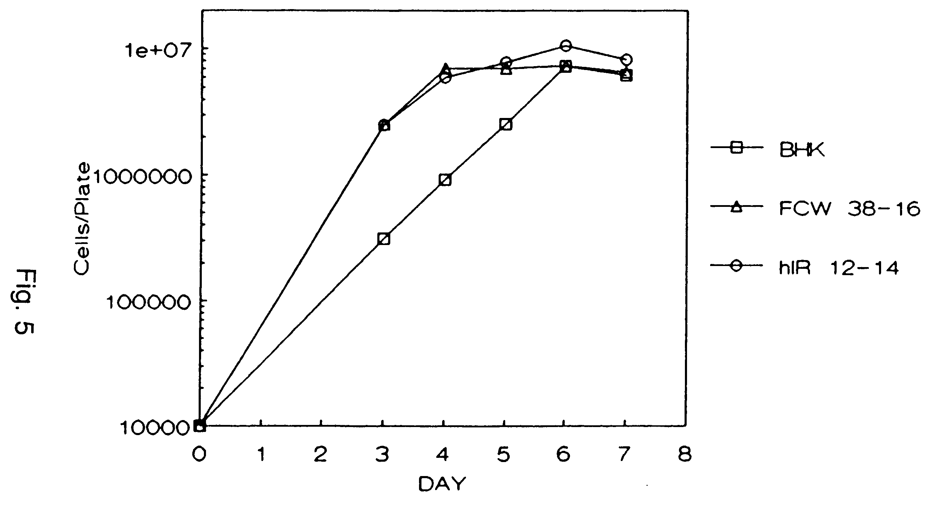

The growth curves for the three different cell lines in FCS-containing medium are shown in Fig. 5. Based on the counts from the first three days, the cell lines FCW 38-16 and hIR 12-14 show a doubling time of 9 hrs as opposed to 16 hrs for BHK cells.

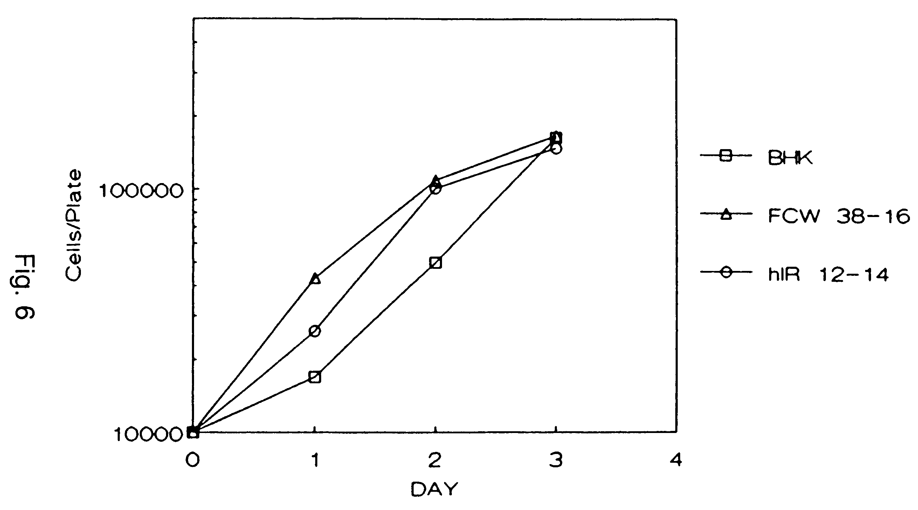

This experiment was repeated the following week, but this time with additional cell counts on day 1 and 2. The growth curves corresponding to the cell counts are shown i Fig. 6.

When figures from the first two days are used to calculate the generation time, FCW 38-16 and hIR 12-14 double each 14.4 hr and BHK doubles each 20.4 hr. All three cell lines had the same cell counts on day four, and were not counted further.

In the second week of experiment, all three cell lines had longer generation times; the ratio between the generation time for the transformed cell lines and BHK was 1.4 as compared to 1.7 for the first week.

### b) Growth in serum-free medium supplemented with human insulin

Three growth experiments with all three cell lines in serum-free medium supplemented with human insulin were performed over three weeks, and cell counts from these three experiments were averaged to generate the growth curves shown in Fig. 6.

BHK cells grown without serum and without added human insulin were counted for one week.

FCW 38-16 consistently grew to higher densities under these conditions, but ultimately died on day 8 - 9.

hIR 12-14 formed grape-like clusters of loosely attached cells, forming many floating cells that stayed alive for 2 - 3 days.

### Conclusion

In two independent growth-curve experiments in serum supplemented with 5% FCS, the transformed cell lines showed a shorter generation time than BHK cells. At the end of each experiment all three cell lines showed the same cell density on the plates.

Although the generation times obtained in both weeks of analysis are not the same, it may be concluded that the growth curves support the observed difference in growth rate of FCW 38-16 (and hIR 12-14) compared to BHK.

Under serum-free conditions (supplemented with insulin), the FCW 38-16 cell line performed far better than both the BHK and hIR 12-14 cell lines, supporting the utility of cells of the present invention expressing a hybrid insulin/IGF-I receptor.

## Claims

1. A mammalian cell containing a hybrid DNA insert which comprises a first DNA sequence encoding part of the extracellular domain of the insulin receptor and a second DNA sequence encoding part of the extracellular domain of insulin-like growth factor (IGF) receptor, said cell having improved growth properties in serum-free culture media compared to cells which do not contain said hybrid DNA insert.

2. A cell according to claim 1, wherein a DNA sequence encoding exon 2, or a fragment thereof, of the insulin receptor is replaced by a DNA sequence encoding exon 2, or a fragment thereof, of the IGF receptor.

3. A cell according to claim 1, wherein a DNA sequence encoding exon 3, or a fragment thereof, of the insulin receptor is replaced by a DNA sequence encoding exon 3, or a fragment thereof, of the IGF receptor.

4. A cell according to claim 1, wherein a DNA sequence encoding exons 2 and 3, or a fragment thereof, of the insulin receptor is replaced by a DNA sequence encoding exons 2 and 3, or a fragment thereof, of the IGF receptor.

5. A cell according to claim 1, wherein a DNA sequence encoding exon 2, or a fragment thereof, of the IGF receptor is replaced by a DNA sequence encoding exon 2, or a fragment thereof, of the insulin receptor.

6. A cell according to claim 1, wherein a DNA sequence encoding exon 3, or a fragment thereof, of the IGF receptor is replaced by a DNA sequence encoding exon 3, or a fragment thereof, of the insulin receptor.

7. A cell according to claim 1, wherein a DNA sequence encoding exons 2 and 3, or a fragment thereof, of the IGF receptor is replaced by a DNA sequence encoding exons 2 and 3, or a fragment thereof, of the insulin receptor.

8. A cell according to claim 4, wherein the DNA insert has the partial DNA sequence shown in Fig. 4A-4F (encoding the extracellular domain of the hybrid insulin/IGF-I receptor), or a suitable modification thereof.

9. A cell according to any of claims 1-8, which further contains an inserted DNA sequence encoding a desired polypeptide.

10. A process for producing a desired polypeptide, which comprises culturing a cell according to claim 9 in a suitable nutrient medium under conditions conducive to the expression of the polypeptide and recovering the polypeptide from the culture.

11. A process according to claim 10, wherein the medium is a serum-free medium.

## Patentansprüche

1. Eine Säugerzelle, die ein hybrides DNA-Insert enthält, das eine erste DNA-Sequenz, die einen Teil der extrazellulären Domäne des Insulinrezeptors kodiert, und eine zweite DNA-Sequenz umfaßt, die einen Teil der extrazellulären Domäne des Rezeptors für Insulin-ähnlichen Wachstumsfaktor (IGF) kodiert, wobei besagte Zelle verbesserte Wachstumseigenschaften in serumfreien Kulturmedien besitzt, verglichen mit Zellen, die besagtes hybrides DNA-Insert nicht enthalten.

2. Eine Zelle nach Anspruch 1, wobei eine DNA-Sequenz, die Exon 2, oder ein Fragment davon, des Insulinrezeptors kodiert, ersetzt ist durch eine DNA-Sequenz, die Exon 2, oder ein Fragment davon, des IGF-Rezeptors kodiert.

3. Eine Zelle nach Anspruch 1, wobei eine DNA-Sequenz, die Exon 3, oder ein Fragment davon, des Insulinrezeptors kodiert, ersetzt ist durch eine DNA-Sequenz, die Exon 3, oder ein Fragment davon, des IGF-Rezeptors kodiert.

4. Eine Zelle nach Anspruch 1, wobei eine DNA-Sequenz, die die Exons 2 und 3, oder ein Fragment davon, des Insulinrezeptors kodiert, ersetzt ist durch eine DNA-Sequenz, die die Exons 2 und 3, oder ein Fragment davon, des IGF-Rezeptors kodiert.

5. Eine Zelle nach Anspruch 1, wobei eine DNA-Sequenz, die Exon 2, oder ein Fragment davon, des IGF-Rezeptors kodiert, ersetzt ist durch eine DNA-Sequenz, die Exon 2, oder ein Fragment davon, des Insulinrezeptors kodiert.

6. Eine Zelle nach Anspruch 1, wobei eine DNA-Sequenz, die Exon 3, oder ein Fragment davon, des IGF-Rezeptors kodiert, ersetzt ist durch eine DNA-Sequenz, die Exon 3, oder ein Fragment davon, des Insulinrezeptors kodiert.

7. Eine Zelle nach Anspruch 1, wobei eine DNA-Sequenz, die die Exons 2 und 3, oder ein Fragment davon, des IGF-Rezeptors kodiert, ersetzt ist durch eine DNA-Sequenz, die die Exons 2 und 3, oder ein Fragment davon, des Insulinrezeptors kodiert.

8. Eine Zelle nach Anspruch 4, wobei das DNA-Insert die partielle DNA-Sequenz besitzt, die in Fig. 4A-4F dargestellt ist (die die extrazelluläre Domäne des hybriden Insulin-/IGF-I-Rezeptors kodiert), oder eine geeignete Modifikation davon.

9. Eine Zelle nach einem der Ansprüche 1-8, die weiterhin eine inserierte DNA-Sequenz enthält, die ein gewünschtes Polypeptid kodiert.

10. Ein Verfahren zur Herstellung eines gewünschten Polypeptids, das Kultivieren einer Zelle nach Anspruch 9 in einem geeigneten Nährstoffmedium unter Bedingungen, die für die Expression des Polypeptids geeignet sind, und Gewinnen des Polypeptids aus der Kultur umfaßt.

11. Ein Verfahren nach Anspruch 10, wobei das Medium ein serumfreies Medium ist.

## Revendications

1. Cellule mammifère contenant un insert d'ADN hybride qui comprend une première séquence d'ADN codant pour une partie du domaine extracellulaire du récepteur de l'insuline et une seconde séquence d'ADN codant pour une partie du domaine extracellulaire du récepteur du facteur de croissance de type insuline (IGF), ladite cellule ayant des propriétés de croissance améliorées dans des milieux de culture sans sérum par comparaison à des cellules qui ne contiennent pas ledit insert d'ADN hybride.

2. Cellule selon la revendication 1, dans laquelle une séquence d'ADN codant pour l'exon 2 du récepteur de l'insuline ou pour un de ses fragments est remplacée par une séquence d'ADN codant pour l'exon 2 du récepteur de l'IGF ou pour un de ses fragments.

3. Cellule selon la revendication 1, dans laquelle une séquence d'ADN codant pour l'exon 3 du récepteur de l'insuline ou pour un de ses fragments est remplacée par une séquence d'ADN codant pour l'exon 3 du récepteur de l'IGF ou pour un de ses fragments.

4. Cellule selon la revendication 1, dans laquelle une séquence d'ADN codant pour les exons 2 et 3 du récepteur de l'insuline ou pour un de leurs fragments est remplacée par une séquence d'ADN codant pour les exons 2 et 3 du récepteur de l'IGF ou pour un de leurs fragments.

5. Cellule selon la revendication 1, dans laquelle une séquence d'ADN codant pour l'exon 2 du récepteur de l'IGF ou pour un de ses fragments est remplacée par une séquence d'ADN codant pour l'exon 2 du récepteur de l'insuline ou pour un de ses fragments.

6. Cellule selon la revendication 1, dans laquelle une séquence d'ADN codant pour l'exon 3 du récepteur de l'IGF ou pour un de ses fragments est remplacée par une séquence d'ADN codant pour l'exon 3 du récepteur de l'insuline ou pour un de ses fragments.

7. Cellule selon la revendication 1, dans laquelle une séquence d'ADN codant pour les exons 2 et 3 du récepteur de l'IGF ou pour un de leurs fragments est remplacée par une séquence d'ADN codant pour les exons 2 et 3 du récepteur de l'insuline ou pour un de leurs fragments.

8. Cellule selon la revendication 4, dans laquelle l'insert d'ADN a la séquence d'ADN partielle présentée dans la figure 4A-4F (codant pour le domaine extracellulaire du récepteur hybride d'insuline/IGF-I), ou une de ses modifications appropriées.

9. Cellule selon l'une quelconque des revendications 1 - 8, dans laquelle est insérée en outre une séquence d'ADN codant pour un polypeptide désiré.

10. Procédé de production d'un polypeptide désiré, qui comprend la culture d'une cellule selon la revendication 9 dans un milieu nutritif convenable dans des conditions qui conduisent à l'expression du polypeptide, et la récupération du polypeptide à partir de la culture.

11. Procédé selon la revendication 10, dans lequel le milieu est un milieu sans sérum.
